## Europäisches Patentamt
## European Patent Office
## Office européen des brevets

(19)

(11) Veröffentlichungsnummer: **0 017 176**
**B1**

(12)

# EUROPÄISCHE PATENTSCHRIFT

(45) Veröffentlichungstag der Patentschrift:
**06.07.83**

(51) Int. Cl.³: **C 12 N 11/14**

(21) Anmeldenummer: **80101640.3**

(22) Anmeldetag: **27.03.80**

(86) Internationale Anmeldenummer:
**PCT/7 /**

(54) **Verfahren zur Immobilisierung von enzymatisch aktiven Präparaten.**

(30) Priorität: **30.03.79 DE 2912827**

(43) Veröffentlichungstag der Anmeldung:
**15.10.80 Patentblatt 80/21**

(45) Bekanntmachung des Hinweises auf die Patenterteilung:
**06.07.83 Patentblatt 83/27**

(84) Benannte Vertragsstaaten:
**AT BE CH DE FR GB IT LU NL SE**

(56) Entgegenhaltungen:
**DE-A-1 939 347**
**DE-A-2 103 243**
**DE-C-536 546**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft,
Carl-Bosch-Strasse 38, D-6700 Ludwigshafen (DE)**

(72) Erfinder: **Meissner, Ruprecht, Haardstrasse 4,
D-6800 Mannheim 1 (DE)**
Erfinder: **Merz, Gerhard, Dr., Carl-Bosch-Ring 43,
D-6710 Frankenthal (DE)**
Erfinder: **Klefenz, Heinrich, Dr., Schillerstrasse 10,
D-6701 Hochdorf-Assenheim 2 (DE)**
Erfinder: **Zahn, Wolfgang, Dr., Parkstrasse 6,
D-6701 Altrip (DE)**

Verfahren zur Immobilisierung von enzymatisch aktiven Präparaten

Es ist bereits bekannt, Enzyme bzw. Biomassen zu immobilisieren. Mit solchen Immobilisaten lassen sich Prozesse wirtschaftlich und verfahrenstechnisch günstiger durchführen. Zur Immobilisierung werden meist organische Materialien herangezogen, wie Cellulosederivate, organische Polymere — z. B. Polyphenole — oder Ionen-Austauschharze. Auch Kombinationen von organischen und anorganischen Trägermaterialien, z. B. Acrylate mit Molekularsieben oder Aktivkohle (DE-A-2 703 834) sind für diesen Zweck vorgeschlagen worden. Außerdem sind einige Verfahren beschrieben, die einen anorganischen Träger verwenden, z. B. Aluminiumoxid (DE-A-2 537 670), kolloidale Kieselsäure (DE-A-2 713 861) und Kieselgele (DE-A-1 939 347).

Die Herstellung dieser Immobilisate ist jedoch recht umständlich und aufwendig, oder die in diesen enthaltenen Biokatalysatoren sind nicht sehr aktiv. Weiter bereitet die Formgebung Schwierigkeiten.

Es wurde nun ein Verfahren gefunden, bei dem diese Nachteile nicht auftreten.

Gegenstand der Erfindung ist ein Verfahren zur Immobilisierung von enzymatisch aktiven Präparaten, welches darin besteht, daß man eine Fällungskieselsäure oder Kieselsäure-Hydrogel enthaltende Suspension herstellt, in diese einen porösen Füllstoff und die enzymatisch aktive Substanz einarbeitet und die so erhaltene Masse in Formkörper überführt und trocknet.

Die Fällungskieselsäure kann nach bekannten Verfahren hergestellt werden (vgl. DE-C-536 546). So kann man Kieselsäure aus Wasserglas mit Schwefelsäure ausfällen. Die Kieselsäure wird anschließend beispielsweise in einer Filterpresse salzfrei gewaschen und in einer Mühle, z. B. einer Zahnkolloidmühle, so weit zerkleinert, bis eine fließfähige Suspension von Kieselsäurepartikeln in Wasser entstanden ist. Es kann hierbei zweckmäßig oder erforderlich sein, vor, während oder nach der Mahlung Wasser zuzugeben.

Anstelle einer Fällungskieselsäure kann auch ein nach bekannten Verfahren hergestelltes Kieselsäure-Hydrogel (vgl. z. B. DE-B-2 103 243) verwendet werden, das je nach dem zu verwendenden Enzympräparat als eng-, mittel- oder weitporig ausgebildet ist und gegebenenfalls salzfrei gewaschen wurde.

Unter Suspension wird ein grobdisperses System verstanden, bei dem die in einem flüssigen Lösungsmittel verteilten Partikel einen Durchmesser besitzen, der im wesentlichen größer als 1 μm ist (vgl. Hollemann-Wiberg, Lehrbuch der anorganischen Chemie, 57—70 Auflage, 1964, Seite 336). Die Konsistenz der Suspension kann in weiten Grenzen von dünnflüssig bis pastös variieren.

Als Suspensionsmittel wird in der Regel Wasser verwendet. Es können aber auch andere Flüssigkeiten oder deren Mischungen untereinander oder mit Wasser verwendet werden, sofern diese das Enzympräparat nicht schädigen.

In die Kieselsäure-Suspension wird — je nach Viskosität mit einem Rührwerk oder einem Kneter — der poröse Füllstoff eingearbeitet. Der Füllstoff hat die Aufgabe, den fertigen Katalysator wasserfest zu machen, eine ausreichende Diffusion zu gewährleisten und enzymhaltigen Biomassen die Möglichkeit des Quellens zu geben, ohne daß der Katalysator zerstört wird. Als Füllstoffe eignen sich z. B. Aktivkohle, Kieselgur, Ruß und Kieselgel. Art und Menge des Füllstoffes richtet sich nach der Art und Menge des einzuarbeitenden Enzympräparates.

Nach der Zugabe des Füllstoffes wird das — in der Regel als Feuchtmasse — vorbereitete Enzympräparat ebenfalls mechanisch in die erhaltene Masse eingearbeitet.

Anschließend wird das so gewonnene Produkt durch Stehenlassen oder Zugabe eines Elektrolyten, z. B. Calciumcarbonat, verfestigt.

Die verfestigte Masse wird danach in Formkörper überführt, was z. B. durch Herstellen von Extrudaten mit einer Strangpresse geschehen kann. So können selbst Strangpreßlinge mit kleinen Abmessungen, z. B. 0,5 mm Durchmesser, hergestellt werden.

Die Formkörper werden schließlich getrocknet. Dabei muß die Temperatur so gewählt werden, daß das Enzympräparat nicht irreversibel inaktiviert wird.

Das neue Verfahren läßt sich mit Enzymen und Enzympräparaten, wie Zellmaterial, mikrobiologischen, pflanzlichen, tierischen oder humanen Ursprungs durchführen. Beispiele hierfür sind Escherichia coli, Saccharomyces cerevisiae und Arthrobacter sp. Der Gehalt des Biokatalysators an Trockenzellmasse kann dabei bis zu 30 Gewichtsprozent betragen.

Die Erfindung führt im Vergleich mit dem Stand der Technik zu folgenden Vorteilen:

Das enzymatisch aktive Präparat kann im neutralen pH-Bereich in die Trägermasse eingearbeitet werden, die in diesem Bereich lange Zeit stabil ist.

Das Trägermaterial kann vor der Einarbeitung des Enzyms auf einfache Weise salzfrei gewaschen werden.

Das Verfahrensprodukt kann in beliebige Formkörper überführt werden.

Das Verfahrensprodukt kann leicht bis auf einen Gehalt an Restwasser getrocknet werden, der dem Enzym nicht mehr schadet.

Das immobilisierte enzymatisch aktive Präparat kann in dem Träger quellen, ohne daß der Träger mitquillt oder sich verformt.

Das Verfahrensprodukt ist sehr druckfest, so daß es auch in hohen Säulen eingesetzt werden kann.

Aufgrund seiner hohen Porosität werden mit dem Verfahrensprodukt hohe Diffusionskoeffizienten erreicht, ohne daß eine Auswaschung des Enzympräparates erfolgt.

Das Verfahrensprodukt behält seine enzymatische Aktivität über einen sehr langen Zeitraum bei.

Zur Immobilisierung der enzymatischen aktiven Präparate werden toxikologisch unbedenkliche Substanzen verwendet, die gegen den Befall von Mikroorganismen resistent und gegen den Angriff von Chemikalien stabil sind.

### Beispiel 1

Als Trägermaterial wird eine aus Na-Wasserglas und Schwefelsäure hergestellte und in einer Filterpresse salzfrei gewaschene Fällungskieselsäure verwendet (DE-C-536 546, Beispiel 1).

Die Fällungskieselsäure-Paste mit ca. 23% $SiO_2$ wird mit 4,5 Gewichtsprozent Wasser versetzt und in einer Zahnkolloidmühle zu einer Suspension vermahlen. Die Teilchengröße liegt zu mindestens 95% über 1 μm.

In 735 g der dünnflüssigen Fällungskieselsäure-Suspension mit einem $SiO_2$-Gehalt von ca. 22% werden mit einem Laborrührwerk 75 g eines weitporigen Kieselsäure-Xerogels mit einer Korngröße von ca. 5 bis 20 μm eingerührt.

Die Mischung soll einen pH-Wert von ca. 5 aufweisen. Wenn erforderlich, wird der pH-Wert mit verdünnter Schwefelsäure oder verdünnter Natronlauge nachgestellt.

In diese Mischung werden 187,5 g einer Trockenhefeanschlämmung (Saccharomyces cerevisiae) mit einem Feststoffgehalt von ca. 40% und einem pH-Wert von 6 eingerührt.

Nach ca. einstündigem Stehen wird die Katalysatormasse auf einer Strangpresse zu Extrudaten mit 2,5 mm Durchmesser verpreßt und anschließend 24 Stunden bei 55° C in einem Trockenschrank getrocknet.

Nach dem Trocknen ist der Strangdurchmesser auf ca. 1,5 mm geschrumpft.

1 g des so erhaltenen immobilisierten Enzyms hydrolisiert pro Stunde 6,4 g Saccharose. Das entspricht einer Enzymaktivität von 68,7%, wenn man die Enzymaktivität einer identischen Menge nicht immobilisierten Zellmaterials gleich 100% setzt.

### Beispiel 2

Als Trägermaterial dient ein aus Na-Wasserglas und Schwefelsäure hergestelltes weitporiges granulatförmiges Kieselsäure-Hydrogel, das salzfrei gewaschen wurde (DE-C-456 406, Beispiel 2).

Das Hydrogel mit einem $SiO_2$-Gehalt von ca. 14% wird unter Zusatz von Wasser in einer Zahnkolloidmühle zu einer 13prozentigen Hydrogel-Suspension vermahlen. Die Teilchengröße liegt zu mindestens 95% über 1 μm.

In 2000 g der Hydrogel-Suspension werden mit einem Laborrührwerk 130 g Kieselgur eingerührt. In diese Mischung werden 390 g Hefeanschlämmung (Saccharomyces cerevisiae) mit einem Trockenhefegehalt von ca. 33% eingearbeitet. Zur Beschleunigung der Verfestigung der Katalysatormasse werden als Elektrolyt 40 g einer 40prozentigen $CaCO_3$-Anschlämmung zugegeben. Anschließend wird die verfestigte Katalysatormasse auf einer Strangpresse zu 0,8 mm Extrudaten verpreßt und in einem Trockenschrank 24 Stunden bei 55° C getrocknet.

Der nach der Trocknung durch Schrumpfen erhaltene Strangdurchmesser beträgt ca. 0,5 mm.

1 g des so erhaltenen immobilisierten Enzyms hydrolisiert pro Stunde 6,8 g Saccharose. Das entspricht einer Enzymaktivität von 78,7% im Vergleich zur Enzymaktivität der nicht immobilisierten Zellmasse, die gleich 100% gesetzt wird.

### Beispiel 3

Es werden in 970 g einer wie unter Beispiel 2 hergestellten Hydrogel-Suspension 60 g Kieselgur mit einem Laborrührwerk eingerührt. In diese Mischung werden 240 g E. coli ATCC 11105 mit einem Trockensubstanzanteil von ca. 27% eingearbeitet. Während weiteren Rührens erfolgt die Verfestigung der Katalysatormasse unter Granulierung.

Anschließend wird das granulierte Material in einem Trockenschrank 24 Stunden bei 55° C unter Vakuum getrocknet. Der mittlere Korndurchmesser des getrockneten Katalysators beträgt ca. 2 mm.

1 g des so erhaltenen immobilisierten Enzyms hydrolysiert pro Minute 5,1 μMol Penicillin G unter Bildung von 6-Aminopenicillansäure. Das entspricht einer Enzymaktivität von 10,5% im Vergleich zur Enzymaktivität der nicht immobilisierten Zellmasse, die gleich 100% gesetzt wird.

### Beispiel 4

Es werden in 920 g einer wie unter Beispiel 2 hergestellten Hydrogel-Suspension 60 g Kieselgur mit einem Laborrührwerk eingerührt. In diese Mischung werden 300 g Arthrobacter NRRL 3726 mit einem Trockensubstanzanteil von ca. 19% eingearbeitet. Zur Beschleunigung der Verfestigung der Katalysatormasse werden als Elektrolyt 30 g einer 40prozentigen $CaCO_3$-Anschlämmung zugegeben. Anschließend wird die verfestigte Katalysatormasse auf einer Strangpresse zu Extrudaten mit 1,5 mm Durchmesser verpreßt und in einem Trockenschrank 24 Stunden bei 55° C unter Vakuum getrocknet. Der nach der Trocknung erhaltene Strangdurchmesser beträgt ca. 1 mm.

1 g des so erhaltenen immobilisierten Zellmaterials isomerisiert 19 μMol Glucose zu Fructose pro Minute. Das entspricht einer Enzymaktivität von 56,4% im Vergleich zur Enzymaktivität der nicht immobilisierten Zellmasse, die gleich 100% gesetzt wird.

Die in den vorgenannten Beispielen erwähnten Mikroorganismen können von den angegebenen Hinterlegungsstellen frei bezogen werden. Sollten diese irgendwann nicht mehr zur Verfügung stehen, können die Beispiele mit anderen Mikroorganismen, die enzymatische Aktivität besitzen, durchgeführt werden.

**Patentanspruch**

Verfahren zur Immobilisierung von enzymatisch aktiven Präparaten, dadurch gekennzeichnet, daß man eine Fällungskieselsäure oder Kieselsäure-Hydrogel enthaltende Suspension herstellt und anschließend in diese einen porösen Füllstoff und das enzymatisch aktive Präparat einarbeitet und die erhaltene Masse in Formkörper überführt und trocknet.

**Claim**

A process for immobilizing enzymatically active formulations, wherein a suspension containing precipitated silica or silica hydrogel is prepared, and then a porous filler and the enzymatically active formulation are incorporated into the suspension, and the resulting composition is moulded and dried.

**Revendication**

Procédé d'immobilisation de préparation à activité enzymatique, caractérisé par le fait qu'on prépare une suspension contenant de l'acide silicique précipitée ou de l'hydrogel d'acide silicique, puis on incorpore dans cette suspension une charge poreuse et la préparation à activité enzymatique et l'on transforme la masse obtenue en corps moulé que l'on sèche.